# EUROPÄISCHE PATENTANMELDUNG

(11) **EP 1 792 637 A2**
(43) Veröffentlichungstag der Anmeldung: **06.06.2007**
(21) Anmeldenummer: 06021932.6
(22) Anmeldetag: 19.10.2006
(51) Int. Cl.: A61M 25/00

(54) **Mehrlumiges Kathetersystem sowie Verfahren zu dessen Herstellung**

(30) Priorität: 26.10.2005 DE 102005051211
(71) Anmelder: Bionic Medizintechnik GmbH, 61381 Friedrichsdorf (DE)
(72) Erfinder: Jungmann, Ralf, 63694 Limeshain (DE)
(74) Vertreter: Schubert, Siegmar

(57) **Zusammenfassung**

In einem zweilumigen Kathetersystem erstrecken sich zwei von je einer flexiblen Wand umgebene innere Leitungen durchgängig von einschließlich zwei länglichen Anschlußabschnitten (3, 4) an einem proximalen Ende des Kathetersystems bis in jeweils wenigstens eine Öffnung (16, 16a) an einem distalen Ende des Kathetersystems erstrecken. Um eine flexible Herstellung eines solchen Kathetersystems mit geringem Materialabfall und eine schonende Anwendung an einem Patienten zu erreichen, sind die beiden Leitungen in je einem Einzelkatheter (1, 2) ausgebildet und die beiden Einzelkatheter (1, 2) sind über einen gemeinsamen zentralen Längenabschnitt (11, 11a) mittels eines durchgängigen Verbindungselements an ihren einander zugewandten Außenseiten (19, 20) miteinander verbunden. Der zentrale Längenabschnitt (11, 11a) erstreckt sich zwischen den beiden länglichen Anschlußabschnitten (3, 4) an dem proximalen Ende einerseits und der Öffnung (16a) an dem distalen Ende des einen (2) der beiden Einzelkatheter andererseits, über welchen ein länglicher Endabschnitt (15) des anderen (1) der beiden Einzelkatheter hinausragt.

## Beschreibung

Die Erfindung betrifft ein mehrlumiges Kathetersystem nach dem Oberbegriff des Anspruchs 1 bzw. des nebengeordneten Anspruchs 9 und Verfahren zu dessen Herstellung nach den Ansprüchen 7, 8 bzw. 16, 17.

Bekannte Katheter können aus einem durchgehenden, einlumigen, weichen Schlauch aus inertem Silikon-Elastomer, der sich einem Gefäßverlauf eines Patienten anpaßt und bis in einen Herzvorhof vorgeschoben werden kann, bestehen. Eine fischmaul-förmige Katheterspitze, der unmittelbar dahinter zwei Seitenöffnungen zugeordnet sein können, verhindert ein Ansaugen an der Gefäß-Innenwand. Der Katheter umfaßt weiterhin eine Muffe aus Dacron-Gewebe im subkutanen Abschnitt, die den Katheter umschließt und in das Bindegewebe des Patienten zur Fixierung des Katheters einwachsen kann. Für eine Dialyse wird ein solcher Katheter über einen Adapter an ein Dialysegerät angeschlossen (Prospekt "DEMERS-Katheter Implantierbarer Atrium-Katheter für die Dialyse" der Anmelderin). Dieser Katheter ist im wesentlichen problemlos, abgesehen von seiner Dicke, die als die Anwendung erschwerend empfunden werden kann.

Bekannt sind auch doppellumige Katheter zum Anschluß an das menschliche Gefäßsystem, die eine venöse innere Leitung bzw. ein Lumen sowie eine arterielle innere Leitung bzw. Lumen umfassen und an ihrem proximalen Ende mit Anschlußschläuchen aus flexiblem Kunststoff an einer Anschlußstelle bzw. einem Halter verbunden sind (EP 0 453 234 A, DE 202 03 267 A). Die Anschlußschläuche können durch den Halter in eine zu dem Katheter zurückgebogene Konfiguration gebogen sein oder selbst durch entsprechende Formgebung mit Gedächtniseffekt in gebogene bzw. gekrümmte Form gebracht werden. In beiden voranstehenden Fällen sind die Anschlußschläuche mit einem zentralen Längenabschnitt des doppellumigen Katheters an dessen proximalem Ende zu verbinden, was entsprechende Herstellungsschritte erfordert.

Längliche Endabschnitte an dem distalen Ende eines bekannten zweilumigen Katheters können für jedes der beiden Lumen unterschiedliche Wandstärken aufweisen, wodurch die dünnere der beiden Wände bei der Einführung des Katheters in den Patienten zurückweichen kann und so das Einführen erleichtert (EP 0453 234 A). Insbesondere kann der längliche Endabschnitt mit größerem Wanddurchmesser an dem distalen Ende länger sein soll als der Endabschnitt mit dünnerer Wand. Dies setzt jedoch eine spezielle Herstellung des mehrlumigen Katheters voraus.

Zum Stand der Technik gemäß dem Oberbegriff des Anspruchs 1 gehört auch ein mehrlumiger Katheter mit Vielfachleitung, der oberhalb eines sogenannten Teilungspunktes, der sich in einer bestimmten Entfernung zu einem proximalen äußeren Ende des Katheters befindet, wenigstens zwei längliche und verschiedene Endabschnitte an einem distalen äußeren Ende des Katheters aufweist, die sich im wesentlichen parallel zur Längsachse des Katheters über jeweils eine unterschiedliche Länge erstrecken (EP 0 848 662 A). Die Endabschnitte bestehen aus biegsamem Material, welches unter der Einwirkung einer Flüssigkeit, insbesondere einer Körperflüssigkeit, flexibel ist und weisen seitliche Öffnungen oder Kanäle auf, um die Flüssigkeit freizusetzen und/oder zu entnehmen. - Auch dieser mehrlumige Katheter erfordert eine spezielle Herstellung insbesondere zum Auftrennen der länglichen Anschlußabschnitten an dem proximalen Ende sowie der länglichen Endabschnitte an dem distalen Ende, wobei durch Verkürzung des einen der Endabschnitte Material abfällt.

Der vorliegenden Erfindung liegt die Aufgabe zugrunde, ein Kathetersystem mit wenigstens zwei von je einer flexiblen Wand umgebenen inneren Leitungen der gattungsgemäßen Art zu schaffen, das die Nachteile bekannter Katheter vermeidet und sich durch eine flexiblere Herstellung mit geringerem Materialabfall und zuverlässiger, Patienten schonender Anwendbarkeit ermöglicht.

Diese Aufgabe wird für das Kathetersystem in einer ersten Variante nach Anspruch 1 mit den darin angegebenen Merkmalen gelöst und in einer zweiten, herstellungsbedingt bevorzugten Variante nach Anspruch 9 mit dessen Merkmalen.

Entsprechende Verfahren zur Herstellung der Kathetersysteme umfassen die Merkmale der Ansprüche 7, 8 bzw. 16, 17.

Die erfindungsgemäßen Kathetersysteme mit zwei inneren Leitungen bzw. Lumen eignen sich besonders für die kontinuierliche Blutwäsche.

In beiden Varianten wird das mehrlumige erfindungsgemäße Kathetersystem durch Verdoppelung und spezielle Verbindung einzeln gespritzter schlauchförmiger Katheter geschaffen, der bei vergleichsweise geringer Außenabmessungen einen guten Flüssigkeitsaustausch in dem Patienten ermöglicht. Diese Einzelkatheter können in der gewünschten Länge abfallfrei geschnitten sein und somit in abgestuftem Längenverhältnis zusammengesetzt werden, indem sie in einem zentralen Längenabschnitt durchgehend mit einem Verbindungselement an ihren einander zugewandten Seiten miteinander verbunden werden, wozu sie insbesondere gemäß Anspruch 7 bzw. 16 mit einem vorgefertigt extrudierten Verbindungselement oder gemäß Anspruch 8 bzw. 17 unter gleichseitiger Bildung eines Verbindungselements zu einer Einheit verklebt werden. Damit können ohne größere herstellungstechnische Änderungen und Lagerhaltung Varianten des mehrlumigen Kathetersystems ausgeführt werden.

Insbesondere kann das Verbindungselement bei dessen Herstellung als Bogen ausgeformt werden, wenn diese Variante gewünscht wird. Die Mindestlänge des Verbindungselements beträgt insbesondere in dem letztgenannten Fall ca. 50 mm.

Mit dem Verbindungselement wird verhindert, daß die flexiblen Einzelkatheter, die fertigungsgünstig als dünne Schläuche hergestellt werden können, bei stärkerer Biegung kollabieren bzw. knicken. Hierzu kann mit dem Verbindungselement ein Mindestradius bei dem Verlegen des Kathetersystems von ca. 1 cm eingestellt werden.

Indem die Anschlußabschnitte der Einzelkatheter an dem proximalen Ende des Kathetersystems nicht durch das Verbindungselement verbunden sind, können diese ohne weiteres, d.h. ohne Anbringung eines hohlen Verbindungselements zwischen Katheter und speziellen Anschlußleitungen zum Anschluß verwendet werden.

In einer ersten Variante des Kathetersystems nach Anspruch 1 erstreckt sich das Verbindungselement an dem distalen Ende bis zu der Öffnung in der Spitze des einen der beiden Katheter, und ein länglicher Endabschnitt des anderen der beiden Katheter, ebenfalls mit einer Öffnung in der Spitze, ragt frei beweglich über die erstgenannte Spitze hinaus.

In der zweiten Variante des Kathetersystems nach Anspruch 8 erstreckt sich das Verbindungselement mit bis zu zwei freien, d.h. beweglichen Endabschnitten, die sich an den zentralen Längenbereich anschließen, und somit nicht zu deren Spitzen bzw. Öffnungen. Auch hier entfällt die Auftrennung eines Doppellumenelements. Diese zweite Variante hat sich zum zuverlässigen Verkleben des Verbindungselements als besonders vorteilhaft herausgestellt. Die beiden Spitzen mit den Öffnungen an dem distalen Ende des Kathetersystems können in Richtung dessen Längserstreckung auch bei der zweiten Variante gegeneinander versetzt sein, um einen wirkungsvollen Flüssigkeitsaustausch in dem Patienten zu bewirken. Die beiden Spitzen der Einzelkatheter bzw. länglichen Endabschnitte an dem distalen Ende können gemäß Anspruch 6 bzw. 14 in einer fertigungsgünstigen Herstellungsphase fischmaul-förmig ausgeformt werden, um ein Ansaugen an der Gefäß-Innenwand zu verhindern.

Die Weiterbildungen des Kathetersystems eignen sich in der Regel sowohl für die erste als auch die zweite Variante.

Indem das Verbindungselement nach Anspruch 2 bzw. 10 zwei sich über dessen Länge erstreckende gegenüberliegende konkave Seiten aufweist, an denen die einander zugewandten Seiten der beiden Einzelkatheter anliegen, wird eine definierte widerstandsfähige Verbindung zwischen den Einzelkathetern und dem Verbindungselement ermöglicht, ohne Spalt, in dem sich Bakterien entwickeln könnten, und ohne den Außenumfang vergrößernde Umhüllung.

Wenn an dem proximalen Ende des Kathetersystems eine Gewebemanschette zur Festlegung des proximalen Endes an dem Patienten vorgesehen ist, umgibt die Gewebemanschette gemäß Anspruch 3 bzw. 11 die beiden Einzelkatheter, und das die beiden Einzelkatheter verbindende Verbindungselement erstreckt sich zweckmäßig bis in die Gewebemanschette, um eine Infektionsgefahr zu verringern bzw. auszuschließen.

Wesentliche Schritte zur rationellen Herstellung des Kathetersystems nach Anspruch 7 bzw. 8 und Anspruch 16 bzw. 17 wurden bereits weiter oben erwähnt. Sie umfassen im wesentlichen die Extrusion bzw. das Spritzen der einzelnen Schläuche sowie deren Verbindung durch Verkleben mit dem ebenfalls extrudierten Verbindungselement bzw. die Bildung eines durchgängigen Verbindungselements durch Verkleben der beiden Einzelkatheter an ihren einander zugewandten Enden in der Art eines Ausfugens mit einer Kunststoffmasse, insbesondere Zwischenspritzens eines Silikon-Elastomers zwischen die beiden Einzelkatheter, wobei die Länge des durchgängigen Verbindungselements gleich derjenigen des gemeinsamen zentralen Längenabschnitts der beiden Katheter zwischen den Endpunkten dieses Längenabschnitts ist. Die Formgebung der Einzelkatheter, insbesondere an deren Spitzen bzw. Endabschnitten an dem distalen Ende, kann bereits vor dem Verkleben der beiden Einzelkatheter erfolgen.

Die Erfindung wird im folgenden anhand einer Zeichnung mit sechs Figuren erläutert, aus denen weitere wesentliche Einzelheiten hervorgehen können.
Es zeigen:
- Figur 1: eine erste Variante eines zweilumigen Kathetersystems in einer Seitenansicht,
- Figur 2: einen Querschnitt durch das Kathetersystem gemäß Figur 1 in der darin angedeuteten Schnittebene A-A,
- Figur 3: einen schaubildlichen Teillängsschnitt als Detail B in Figur 1,
- Figur 4: eine zweite Variante eines zweilumigen Katheters in einer Seitenansicht, welche die zum Verkleben bei der Herstellung bevorzugte Ausführungsform ist, und
- Figuren 5 und 6: die den Figuren 2 und 3 entsprechenden, nämlich gleichen Darstellungen der zweiten Variante.

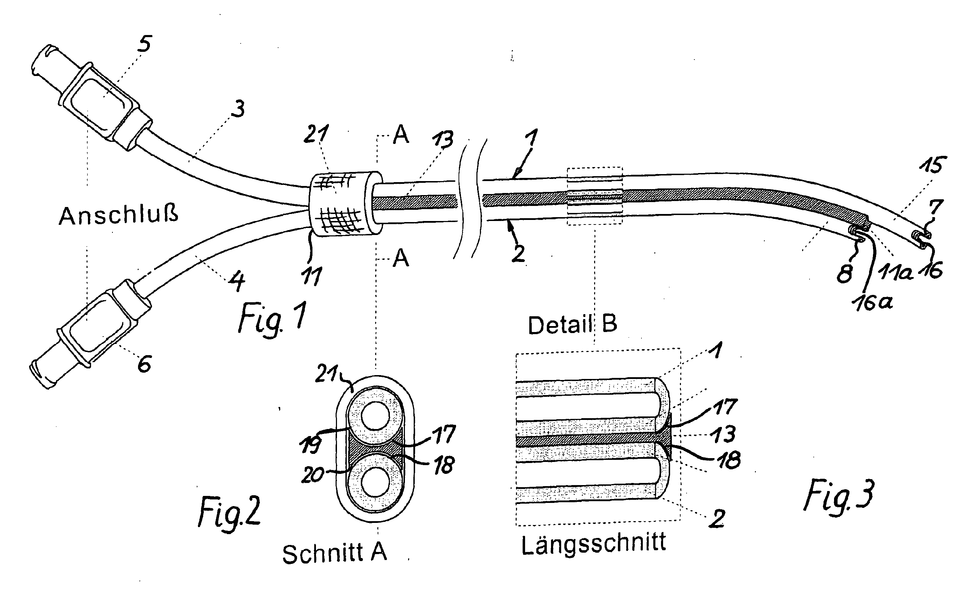

Die Darstellungen in den Figuren 2, 3, 5 und 6 sind vergrößert.

In sämtlichen Figuren sind gleiche Teile mit übereinstimmenden Bezugszeichen versehen.

Für beide in den Figuren 1 bis 6 dargestellten Varianten sind mit 1 und 2 schlauchförmige Einzelkatheter bezeichnet. Jeder der Katheter weist an einem proximalen Ende einen Anschlußabschnitt 3 bzw. 4 auf, der mit einem venösen Anschluß 5 bzw. einem arteriellen Anschluß 6 versehen ist.

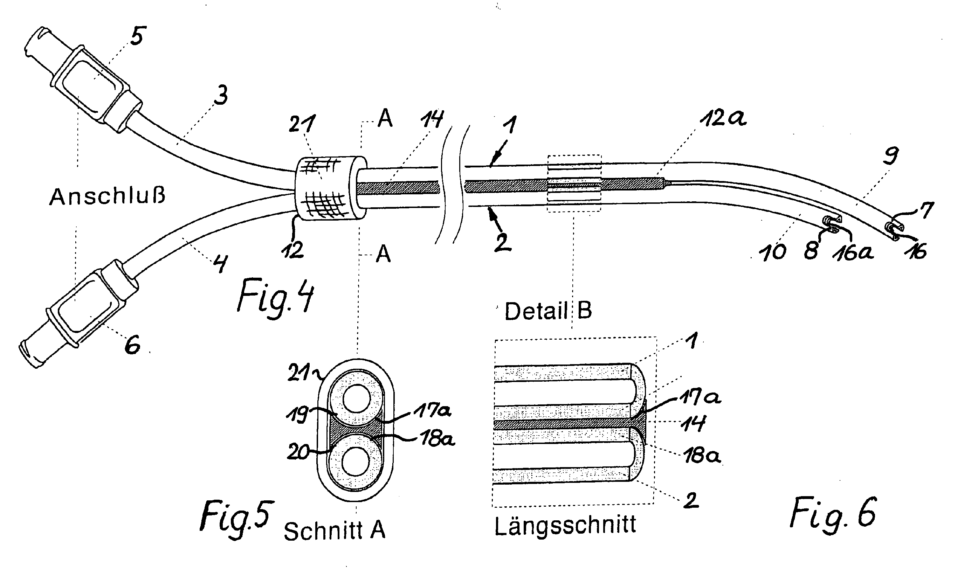

An dem entgegengesetzten Ende, dem distalen Ende, weist jeder der Schläuche eine Spitze 7 bzw. 8 auf, und zwar über der Länge des Katheters versetzt. Die beiden Spitzen 7, 8 sind fischmaul-förmig ausgebildet, wie im einzelnen aus den Figuren 1 und 4 ersichtlich. Die Spitze 7 gehört zu den Endabschnitten 9 und 15 unterschiedlicher Länge bei beiden Varianten. Die Spitze 8 gehört zu dem Endabschnitt 10 nur der zweiten Variante.

Die beiden Einzelkatheter 1, 2 sind über einen zentralen Längenabschnitt, der in der ersten Variante gemäß Figur 1 durch die Endpunkte 11, 11 a definiert ist, bzw. einen kürzeren zentralen Längenabschnitt 12, der in der zweiten Variante nach Figur 4 durch die Endpunkte 12, 12a begrenzt ist, mittels eines länglichen Verbindungselements 13 bzw. 14 miteinander verbunden. Das Verbindungselement 13, 14 bestimmt somit dort, wo er anfängt, die freien Anschlußabschnitte 3, 4 an dem proximalen Ende sowie dort, wo es endet, die beweglichen Endabschnitte 7, 8 an dem distalen Ende der zweiten Variante in Figur 4. In der ersten Variante endet das Verbindungselement 13 an der Spitze 8, und es wird nur ein beweglicher Endabschnitt 15 an der Spitze 7 gebildet.

Dementsprechend sind die beiden Endabschnitte 9, 10 unterschiedlich lang, und zwar gehört der längere Endabschnitt 9 zu dem venösen Einzelkatheter 1 und der kürzere Endabschnitt 10 zu dem arteriellen Einzelkatheter 2 der zweiten Variante.

Bei der gezeichneten Länge des Verbindungselements zwischen den Endpunkten 13, 13a in Figur 1 ist die Länge des Endabschnitts an der Spitze 8 praktisch Null und der Endabschnitt 15 ist verhältnismäßig kurz im Vergleich zu dem entsprechenden Endabschnitt 9 in Figur 4 infolge des in der zweiten Ausführungsform kürzeren Verbindungselements zwischen den Endpunkten 14, 14a.

Wie aus dem Querschnitt in Figur 2 und dem Teillängsschnitt in Figur 3 ersichtlich, weist das Verbindungselement zwei abgewandt gegenüberliegende ähnliche konkave Seiten 17, 18 bzw. 17a, 18a auf, von denen in Figur 3 bzw. 6 jeweils nur eine Hälfte angedeutet ist. An die konkaven Seiten 17, 18 bzw. 17a, 18a schmiegen sich einander zugewandte Seiten 19, 20 des zentralen Längenabschnitts der Einzelkatheter 1, 2 lückenlos an. Die konkaven Seiten des Verbindungselements und die Außenseiten 19, 20 der zentralen Längsabschnitte der Einzelkatheter 1, 2 sind miteinander verklebt.

Die verklebte Gesamtheit der Einzelkatheter 1, 2 und des Verbindungselements 13 bzw. 14, welche jeweils im wesentlichen das Kathetersystem bilden, kann flexibel ausgebildet sein. Wenn gewünscht kann das Verbindungselement 13 bzw. 14 in einem zentralen Teil-Längenbereich gekrümmt vorgeformt sein und diese Form beibehalten bzw. nach Verformung unter Krafteinwirkung wieder annehmen.

Es wird bemerkt, daß sich das Verbindungselement 13 bzw. 14 bis in eine Gewebemanschette 21 erstreckt, um hierin keine Bakterien begünstigende Zwischenräume freizulassen.

Die sich über je einen zentralen Längenabschnitt erstreckenden Verbindungselemente 13, 14 können hinsichtlich ihres proximalen, nicht bezeichneten Endpunktes variabel ausgeführt sein. Wie oben erwähnt, ist die Ausführung gemäß Figur 4 bevorzugt, um eine sichere Verklebung auch an dem Endpunkt 12a zu gewährleisten, wobei die Verbindung ebenfalls bevorzugt durch direktes Verkleben der Einzelkatheter unter Bildung des Verbindungselements 14 erfolgt.

### Bezugszahlenliste

- 1: schlauchförmiger Einzelkatheter (venös)
- 2: schlauchförmiger Einzelkatheter (arteriell)
- 3: Anschlußabschnitt
- 4: Anschlußabschnitt
- 5: venöser Anschluß
- 6: arterieller Anschluß
- 7: Spitze
- 8: Spitze
- 9: Endabschnitt 2. Variante
- 10: Endabschnitt 2. Variante
- 11, 11a: zentraler Längenabschnitt (Endpunkte)
- 12, 12a: zentraler Längenabschnitt (Endpunkte)
- 13: Verbindungselement
- 14: Verbindungselement
- 15: Endabschnitt 1. Variante
- 16, 16a: Öffnungen
- 17, 17a: konkave Seite
- 18, 18a: konkave Seite
- 19: zugewandte Außenseite
- 20: zugewandte Außenseite
- 21: Gewebemanschette
- 22: Anschluß
- 23: Anschluß

## Patentansprüche

1. Zweilumiges Kathetersystem mit zwei von je einer flexiblen Wand umgebenen inneren Leitungen, die sich durchgängig von einschließlich zwei länglichen Anschlußabschnitten (3, 4) an einem proximalen Ende des Kathetersystems bis in jeweils wenigstens eine Öffnung (16, 1 6a) an einem distalen Ende des Kathetersystems erstrecken,
**dadurch gekennzeichnet,**
**daß** die beiden Leitungen in je einem Einzelkatheter (1, 2) ausgebildet sind, daß die beiden Einzelkatheter (1, 2) über einen gemeinsamen zentralen Längenabschnitt (11, 11a) mittels eines durchgängigen Verbindungselements an ihren einander zugewandten Außenseiten (19, 20) miteinander verbunden sind, und daß sich der zentrale Längenabschnitt (11, 11a) zwischen den beiden länglichen Anschlußabschnitten (3, 4) an dem proximalen Ende einerseits und der Öffnung (16a) an dem distalen Ende des einen (2) der beiden Einzelkatheter andererseits erstreckt, über welchen ein länglicher Endabschnitt (15) des anderen (1) der beiden Einzelkatheter hinausragt.

2. Kathetersystem nach Anspruch 1,
**dadurch gekennzeichnet,**
**daß** das Verbindungselement (13) zwei sich über dessen Länge erstreckende, gegenüberliegende konkave Seiten (17, 18) aufweist, an denen die einander zugewandten Außenseiten (19, 20) der beiden Einzelkatheter (1, 2) anliegen.

3. Kathetersystem nach Anspruch 1 oder 2,
**dadurch gekennzeichnet,**
**daß** an dem proximalen Ende des Kathetersystems eine Gewebemanschette (21) die beiden Einzelkatheter (1, 2) umgibt und
**daß** sich das die beiden Einzelkatheter (1, 2) verbindende Verbindungselement (13) bis in die Gewebemanschette (21) erstreckt.

4. Kathetersystem nach einem der Ansprüche 1 bis 3,
**dadurch gekennzeichnet,**
**daß** das Verbindungselement (13) mindestens 50 mm lang ist.

5. Kathetersystem nach einem der Ansprüche 1 bis 4,
**dadurch gekennzeichnet,**
**daß** das Verbindungselement (13) vorgebogen ist.

6. Kathetersystem nach einem der Ansprüche 1 - 5,
**dadurch gekennzeichnet,**
**daß** die Einzelkatheter (1, 2) an ihrem distalen Ende fischmaul-förmig enden.

7. Verfahren zur Herstellung eines Kathetersystems mit zwei von je einer flexiblen Wand umgebenen inneren Leitungen, die sich durchgängig von einschließlich zwei länglichen Anschlußabschnitten (3, 4) an einem proximalen Ende des Kathetersystems bis in jeweils wenigstens eine Öffnung (16, 16a) an einem distalen Ende des Kathetersystems erstrecken,
**dadurch gekennzeichnet,**
**daß** zwei aus Kunststoff einzeln extrudierte Einzelkatheter (1, 2) aus flexiblem Material über einen zentralen Längenabschnitt (11, 11a) an ihren einander zugewandten Seiten (19, 20) mit einem extrudierten Verbindungselement (13) zu einer Einheit verklebt werden.

8. Verfahren zur Herstellung eines Kathetersystems mit zwei von je einer flexiblen Wand umgebenen inneren Leitungen, die sich durchgängig von einschließlich zwei länglichen Anschlußabschnitten (3, 4) an einem proximalen Ende des Kathetersystems bis in jeweils wenigstens eine Öffnung (16, 16a) an einem distalen Ende des Kathetersystems erstrecken,
**dadurch gekennzeichnet,**
**daß** zwei aus Kunststoff einzeln extrudierte Einzelkatheter (1, 2) aus flexiblem Material über einen zentralen Längenabschnitt (11, 11a) an ihren einander zugewandten Seiten (19, 20) mit einer Kunststoffmasse verklebt werden, die ein durchgängiges Verbindungselement (13) bildet.

9. Zweilumiges Kathetersystem mit zwei von je einer flexiblen Wand umgebenen inneren Leitungen, die sich durchgängig von einschließlich zwei länglichen Anschlußabschnitten (3, 4) an einem proximalen Ende des Kathetersystems bis in zwei längliche Endabschnitte (9, 10) an einem distalen Ende erstrecken,
**dadurch gekennzeichnet,**
**daß** die beiden Leitungen in je einem Einzelkatheter (1, 2) ausgebildet sind, daß die beiden Einzelkatheter über einen gemeinsamen zentralen Längenabschnitt (12, 12a) zwischen den länglichen Endabschnitten (9, 10) an dem distalen Ende einerseits und den länglichen Anschlußabschnitten (3, 4) an dem proximalen Ende andererseits mittels eines durchgängigen Verbindungselements (14) an ihren einander zugewandten Außenseiten (19, 20) miteinander verbunden sind.

10. Kathetersystem nach Anspruch 9,
**dadurch gekennzeichnet,**
**daß** das Verbindungselement (14) zwei sich über dessen Länge erstreckende, gegenüberliegende konkave Seiten (17a, 18a) aufweist, an denen die einander zugewandten Außenseiten (19, 20) der beiden Einzelkatheter (1, 2) anliegen.

11. Kathetersystem nach Anspruch 9 oder 10,
**dadurch gekennzeichnet,**
**daß** an dem proximalen Ende des Kathetersystems eine Gewebemanschette (21) die beiden Einzelkatheter (1, 2) umgibt, und
**daß** sich das die beiden Einzelkatheter (1, 2) verbindende Verbindungselement (14) bis in die Gewebemanschette (21) erstreckt.

12. Kathetersystem nach einem der Ansprüche 9 bis 11,
**dadurch gekennzeichnet,**
**daß** die länglichen Endabschnitte (9, 10) an dem distalen Ende unterschiedlich lang sind und
**daß** das Verbindungselement (14) vor dem kürzeren (10) der beiden Endabschnitte an dem distalen Ende endet.

13. Kathetersystem nach einem der Ansprüche 9 bis 12,
**dadurch gekennzeichnet,**
**daß** das Verbindungselement (14) mindestens 50 mm lang ist.

14. Kathetersystem nach einem der Ansprüche 9 bis 13,
**dadurch gekennzeichnet,**
**daß** das Verbindungselement (14) vorgebogen ist.

15. Kathetersystem nach einem der Ansprüche 9-14,
**dadurch gekennzeichnet,**
**daß** die Einzelkatheter (1, 2) an ihrem distalen Ende fischmaul-förmig enden.

16. Verfahren zur Herstellung eines Kathetersystems mit zwei von je einer flexiblen Wand umgebenen Leitungen, die sich durchgängig von einschließlich zwei länglichen Anschlußabschnitten (3, 4) an einem proximalen Ende des Kathetersystems bis in zwei längliche Endabschnitte (9, 10) an einem distalen Ende erstrecken,
**dadurch gekennzeichnet,**
**daß** zwei aus Kunststoff einzeln extrudierte Einzelkatheter (1, 2) aus flexiblem Material über eine Länge zwischen den Endabschnitten (9, 10) an dem distalen Ende einerseits und den Anschlußabschnitten (3, 4) an dem proximalen Ende andererseits mit einem extrudierten Verbindungselement (14) an ihren einander zugewandten Außenseiten (19, 20) zu einer Einheit verklebt werden.

17. Verfahren zur Herstellung eines Kathetersystems mit zwei von je einer flexiblen Wand umgebenen Leitungen, die sich durchgängig von einschließlich zwei länglichen Anschlußabschnitten (3, 4) an einem proximalen Ende des Kathetersystems bis in zwei längliche Endabschnitte (9, 10) an einem distalen Ende erstrecken,
**dadurch gekennzeichnet,**
**daß** zwei aus Kunststoff einzeln extrudierte Einzelkatheter (1, 2) aus flexiblem Material über eine Länge zwischen den Endabschnitten (9, 10) an dem distalen Ende einerseits und den Anschlußabschnitten (3, 4) an dem proximalen Ende andererseits mit einer Kunststoffmasse verklebt werden, die ein durchgängiges Verbindungselement (14) bildet.
